# EUROPÄISCHE PATENTSCHRIFT

(11) **EP 0 850 573 B1**
(45) Veröffentlichungstag und Bekanntmachung des Hinweises auf die Patenterteilung: **06.02.2002**
(21) Anmeldenummer: 97810883.5
(22) Anmeldetag: 20.11.1997
(51) Int. Cl.: A41D 19/015, A61F 5/04, A41D 19/01

(54) **Daumenschiene**
Thumb splint
Attelle pour le pouce

(30) Priorität: 23.12.1996 CH 316096
(43) Veröffentlichungstag der Anmeldung: 01.07.1998
(73) Patentinhaber: Olivier Amrein Orthopedie, 1018 Lausanne (CH)
(72) Erfinder: Amrein, Olivier, 1025 St. Sulpice (CH)
(74) Vertreter: Patentanwälte Feldmann & Partner AG

(56) Entgegenhaltungen:
- EP-A- 0 143 348
- DE-A- 3 519 493
- DE-U- 8 806 792
- DE-U- 9 401 355
- US-A- 4 438 532
- US-A- 4 524 464
- US-A- 5 561 856

## Beschreibung

Die vorliegende Erfindung betrifft eine Daumenschiene gemäss Oberbegriff des Patentanspruches 1.

Orthesen für den Daumen, auch Daumenschienen oder Daumenbandagen genannt, werden zur Ruhigstellung des Daumens bei Sehnenverletzungen verwendet. Eine Ruhigstellung ist beispielsweise notwendig, wenn die Sehnen beim Volleyball, Ski- oder Snowboardfahren überdehnt worden ist.

Die bekannten Daumenschienen oder Daumenbandagen sind einstückig geformt (siehe DE-U-9 401 355). Sie bestehen aus einer Daumenhülse zur Aufnahme des Daumens, an welche ein Handrücken- und ein Handflächenteil angeformt ist. Die einzelnen Teile sind entsprechend der Form der Hand dreidimensional vorgeformt. Im Gebrauchszustand wird die Daumenhülse über den verletzten Daumen gestülpt, wobei das Handrückenteil auf den Handrücken und der Handflächenteil auf die Handfläche gelegt wird. Im allgemeinen erstrecken sich diese zwei Teile über die Handwurzel und oft auch teilweise über die Mittelhand. Handflächenteil und Handrückenteil werden mittels eines Klettverschlusses oder mittels Schlaufen gegenseitig festgezogen, so dass die Daumenschiene an der Hand fest anliegt und der Daumen ruhiggestellt ist.

Da die Daumenschiene satt an der Hand anliegen muss, sind die bekannten Ausführungsformen in verschiedenen Grössen erhältlich, damit sie entsprechend der Hand- und Daumengrössen ausgewählt werden können. Dies hat mehrere Nachteile. Erstens ist die Hand oder der Daumen nach einer Verletzung oft geschwollen, so dass die Daumenschiene erst angelegt werden kann, wenn die Schwellung zurückgegangen ist. Zweitens müssen immer wieder Hände bandagiert werden, welche eine Zwischengrösse aufweisen, so dass die eine Daumenschiene zu gross, die nächste Grösse jedoch zu klein ist. Drittens wird die Herstellung verteuert. Ein vierter Nachteil ist, dass stets alle Grössen an Lager gehalten werden müssen, was das Produkt wiederum verteuert.

Es ist deshalb Aufgabe der Erfindung, eine Daumenschiene zu schaffen, die die obengenannten Nachteile behebt.

Diese Aufgabe löst eine Daumenschiene mit den Merkmalen des Patentanspruches 1.

Die erfindungsgemässe Orthese oder Daumenschiene weist eine stufenlos grösseneinstellbare Daumenhülse auf, so dass sie an die Daumen- und Handgrösse anpassbar ist. Die erfindungsgemässe Daumenschiene besteht deshalb aus zwei Schienenteilen, welche je eine offene Daumenschale aufweisen. Die zwei Daumenschalen überlappen sich, so dass eine geschlossenen Hülse zur Aufnahme des Daumens gebildet wird, deren Durchmesser in Abhängigkeit des Ueberlappungsbereiches variiert. Die erfindungsgemässe Daumenschiene und insbesondere die offenen Daumenschalen bestehen aus steifen Abschnitten, welche den Daumen stützen und in seiner Lage fixieren. Sie weisen jedoch auch flexible Abschnitte auf, durch die die Grösseneinstellbarkeit erst möglich wird.

Die erfindungsgemässe Daumenschiene ist über einen weiten Bereich grösseneinstellbar, so dass dieselbe Orthese sowohl für kleine wie auch für Hände einsetzbar ist. Durch die spezielle Lage und Form des Grösseneinstellmittels wird zudem nicht einfach der Durchmesser der Hülse vergrössert, sondern der Winkel des Handflächenteils zu den Daumenschalen wird anatomisch korrekt angepasst. Dies hat nicht nur zur Folge, dass der Daumen optimal stabilisiert wird, sondern gewährleistet auch, dass die restlichen Finger in ihrer Beweglichkeit nicht eingeschränkt werden, so dass die Hand abgesehen vom Daumen nach wie vor einsetzbar ist.

In den Figuren ist ein Ausführungsbeispiel des Erfindungsgegenstandes dargestellt, welches in der nachfolgenden Beschreibung erläutert wird. Es zeigen
- Figur 1: eine perspektivische Darstellung eines ersten Daumenschienenteils;
- Figur 2a: eine perspektivische Darstellung eines zweiten Daumenschienenteils von der Handflächenseite aus gesehen;
- Figur 2b: eine perspektivische Darstellung eines zweiten Daumenschienenteils von der Handrückenseite aus gesehen;
- Figur 3: eine an der Hand angezogene erfindungsgemässe Daumenschiene von der Handflächenseite aus gesehen und
- Figur 4: eine an der Hand angezogene erfindungsgemässe Daumenschiene von der Handrückenseite aus gesehen.

Die erfindungsgemässe Daumenschiene ist zweiteilig geformt und besteht aus einem ersten Schienenteil 1 und einem zweiten Schienenteil 2, welche entsprechend der Form einer Hand vorgeformt sind und steife oder versteifte Teilbereiche aufweisen. Beide Schienenteile 1,2 sind aus handelsüblichen Materialien hergestellt, wie sie für vorgefertigte Bandagen und Daumenschienen verwendet werden. Beispielsweise lassen sich atmungsaktive Kunststoffe verwenden. Flexible und steife Bereiche lassen sich durch Variation der Dicke des Materiales oder durch Verschweissen von zwei oder mehreren Schichten erhalten.

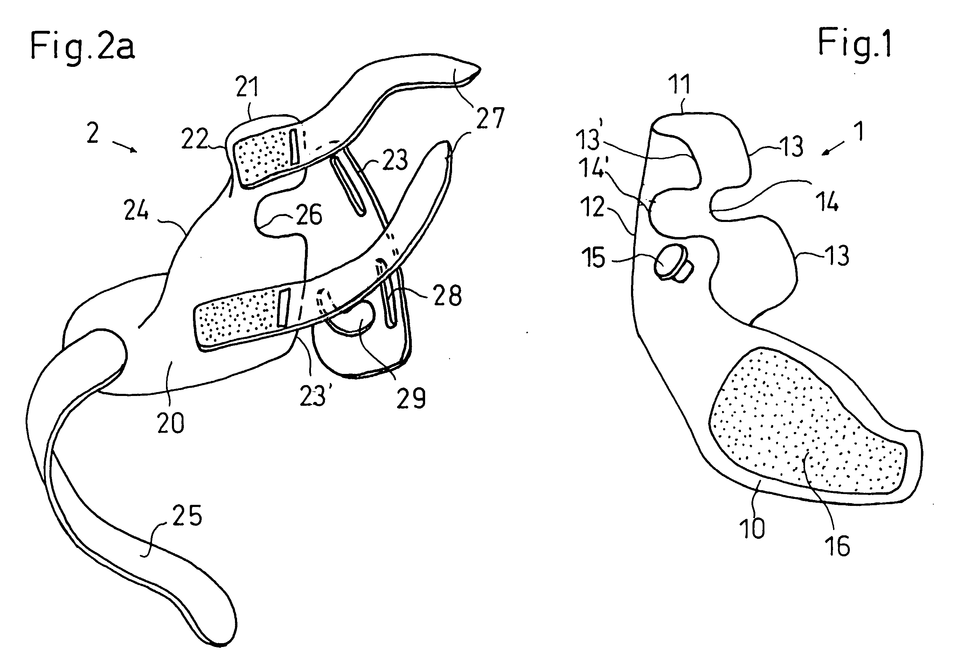

In der Figur 1 ist das erste Schienenteil 1 dargestellt. Es besteht aus einem zungenförmigen, aus flexiblen Material gefertigten Abschnitt, den Handrückenteil 10, und einer daran angeformten, offenen ersten Daumenschale 11. Diese erste Daumenschale 11 bildet im wesentlichen die Verlängerung der breiteren Schmalseite des Handrückenteils 10, wobei sie mit der einen Längsseite des Handrückenteils 10 bündig ist und die andere Längsseite überragt. Die Daumenschale 11 ist gemäss der Figur 1 nach hinten, zur Innenseite hin, gebogen, wobei sie einen Rücken 12 bildet. Der Biegeradius der Daumenschale 11 ist flexibel, zudem ist die Daumenschale 11 im Bereich des Daumenfingerknochens maximal zu einem Viertel ihres Umfanges offen.

Der gebogene Rücken 12 verläuft mindestens annähernd in einem Winkel von 135° zur Hauptausdehnungsrichtung des zungenförmigen Handrückenteils 10, was wie weiter unten gezeigt wird, zur Folge hat, dass der Daumen in leicht gespreizter Haltung von den restlichen Fingern gehalten wird. Der Rücken 12 der ersten Daumenschale 11 ist steif ausgebildet, um die Schalenform zu bilden. Er weist eine leichte Krümmung auf, wobei er zum Handrückenteil hingebogen ist und das Maximum der Krümmung mindestens annähernd in der Mitte liegt. Diese Krümmung ist der Anatomie des zu fixierenden Daumens angepasst. Die Bereiche beidseitig des Rückens 12 sind flexibel gestaltet. So ist die vom Handrückenteil 10 entfernte erste Längskante 13 aus flexiblen Material gefertigt. Sie weist einen ersten Einschnitt 14 auf, welcher sich annähernd bis zum steifen Rücken 12 hin erstreckt und der Erhöhung der Flexibilität dient. An der dem Handrückenteil 10 zugewandten zweiten Längskante 13' ist ebenfalls ein zweiter Einschnitt 14' vorhanden. Er befindet sich in demjenigen Abschnitt der ersten Daumenschale 11, welche das Handrückenteil 10 überragt. Bevorzugterweise ist er versetzt zum ersten Einschnitt 14' und oberhalb diesem angeformt.
Unterhalb des zweiten Einschnittes 14' im Uebergangsbereich vom Handflächenteil 10 zur ersten Daumenschale 11 ist handrückenseitig ein vorstehender Noppen 15 angebracht. Ferner weist das Handrückenteil 10 auf ihrer Aussenseite die eine erste Hälfte eines ersten Befestigungsmittels, hier ein Maschenband 16 eines Klettbandverschlusses, auf.

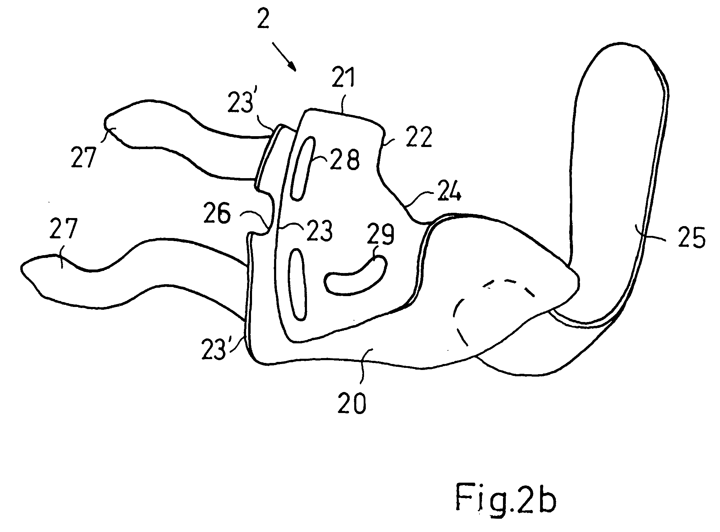

In den Figur 2a und 2b ist das zweite Schienenteil 2 dargestellt. Es ist ähnlich einem einseitig ausladenden Trichter geformt, wobei ein erster Abschnitt einen anatomisch vorgeformten Handflächenteil 20 und ein zweiter Abschnitt eine offene zweite Daumenschale 21 mit einem Rücken 22 bilden. Auch der Biegeradius der zweiten Daumenschale 21 ist flexibel gestaltet und der offene Bereich der zweiten Daumenschale 21 entspricht ebenfalls maximal einem Viertel ihres Umfanges.

Die zweite Daumenschale 21 weist einen steifen Rücken 22 auf und ist handrückenseitig ebenfalls steif ausgebildet, wobei sie auf dieser Seite eine steife erste Längskante 23 aufweist. Handflächenseitig ist sie flexibel ausgestaltet bis auf einen Bereich, welcher sich vom Handflächenteil 20 über einen Sattel 24 erstreckt. Die Randbereiche des Handflächenteils 20 sowie eine zweite Längskante 23' der zweiten Daumenschale 21 sind aus flexiblem Material geformt. Am von der Daumenschale 21 abgewandten Ende des Handflächenteils 20 ist eine zweite Hälfte eines ersten Befestigungsmittels, hier ein erstes Klettband 25 angeordnet. An der flexiblen zweiten Längskante 23' der Daumenschale 21 ist ungefähr auf halber Höhe ein Einschnitt 26 vorhanden, welcher die Flexibilität erhöht. Ferner ist mindestens ein zweites Klettband, hier zwei zweite Klettbänder 27, vorhanden. Sie sind ober- beziehungsweise unterhalb des Einschnittes 26 angebracht. Entlang der ersten Längskante 23 der zweiten Daumenschale 21 verläuft mindestens ein, hier zwei Längsschlitze 28, deren Länge mindestens der Breite der zweiten Klettbänder 27 entspricht. Des weiteren ist in diesem Bereich, also handrückenseitig, in der Verlängerung der Sattellinie ein gekrümmter Verstellschlitz 29 vorhanden.

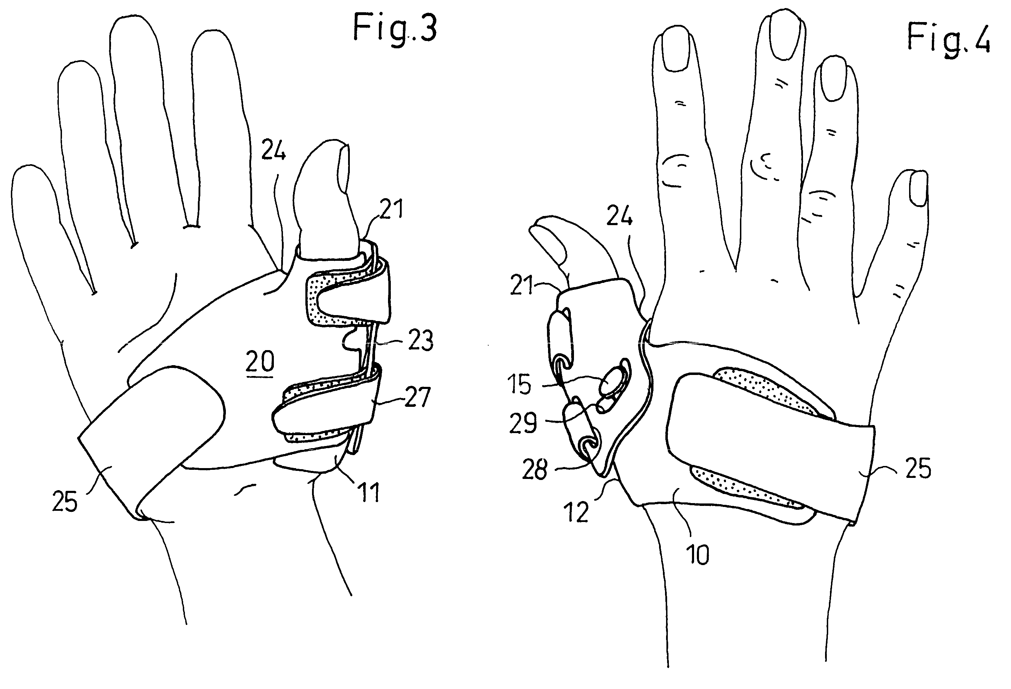

In den Figuren 3 und 4 ist die erfindungsgemässe Daumenschiene im Gebrauchszustand, das heisst angezogen an eine Hand, dargestellt. Die zwei Schienenteile 1,2 sind zusammengefügt, indem der Noppen 15 des ersten Schienenteils 1 durch den Verstellschlitz 29 des zweiten Schienenteils 2 gesteckt ist. Dadurch bilden die erste und die zweite offene Daumenschalen 11,21 eine Daumenhülse, welche auf den Daumen aufsteckbar ist und mindestens den zweiten Fingerknochen sowie den Mittelhandknochen des Daumens aufnimmt. Die zweite Schienenschale 21 umschliesst dabei die erste Schienenschale 11 mindestens teilweise. Der Sattel 24 liegt zwischen Zeigefinger und Daumen auf der Hand auf. Die Oeffnung der ersten Schienenschale 11 ist zum Zeigefinger hingewandt, diejenige der zweiten Schieneschale 21 weist vom Zeigefinger weg. Dadurch stützt der steife Rücken 12 der ersten Daumenschale 11 auf der vom Zeigefinger abgewandten Seite des Daumens und der steife Sattel 24 auf der dem Zeigefinger zugewandten Seite. Auf der Handrückenseite wird der Daumen vom steifen Bereich entlang der ersten Längskante 23 der zweiten Daumenschale 21 fixiert.

Dank des Grösseneinstellmittels, bestehend aus Verstellschlitz 29 und Noppen 15, lässt sich der Ueberlappungsbereich der Daumenschalen 11,21 und somit der Durchmesser der durch die Schalen gebildeten Daumenhülse stufenlos einstellen. Zudem lässt sich auch der Winkel der zwei Schienenteile 1,2 zueinander variieren. Der Sattel 24 gewährleistet, dass der Daumen in einer optimalen Lage, leicht vom Zeigefinger weggespreizt, fixierbar ist. Damit der Daumen in dieser Lage festgehalten wird, wird die Daumenhülse satt anliegend an den Daumen angezogen und mit mindestens einem zweiten Befestigungsmittel, hier die Längsschlitze 28 und die zweiten Klettbänder 27, fixiert. Die zweiten Klettbänder 27 werden hierfür in die Längsschlitze 28 eingeführt und angezogen. Dank der Einschnitte 14,14',26 passen sich die Daumenschalen 11,21 der Form des Daumens an und liegen an diesem stützend an.

Ferner kommt das Handrückenteil 10 auf den Handrücken zu liegen und das Handflächenteil 20 auf die Handfläche. Dabei überdecken diese Teile die Handwurzel und je nach Ausführungsform oder Grösse der Hand gegebenenfalls einen Teil der Mittelhand. Mittels des an diesen Teilen angebrachten ersten Befestigungsmittels, lassen sich Handrücken- und Handflächenteil 10,20 gegenseitig anziehen, so dass die Daumenschiene die Hand satt umschliesst. Das Handflächenteil 20 ist entsprechend einer Handfläche geformt, so dass es, da es ebenfalls steif ausgebildet ist, eine Bewegung des Daumens zur Handfläche hin verhindert.

Die steifen Bereiche dienen somit der Stützung und Stabilisierung des Daumens in einer leicht gespreizten Stellung. Die flexiblen Bereiche dienen der Grösseneinstellung der Daumenschiene und erhöhen den Tragkomfort.

In einer hier nicht dargestellten Ausführungsform ist am Handrücken- und/oder am Handflächenteil eine Handgelenkmanschette angebracht, welche zur Stützung des Handgelenkes dient. Bevorzugterweise ist die Handgelenkmanschette einstückig am Handrücken- und/oder am Handflächenteil angeformt.

## Patentansprüche

1. Daumenschiene zur Ruhigstellung eines Daumens mit einer Hülse zur Aufnahme des Daumens, einem Handrückenteil (10), einem anatomisch vorgeformten Handflächenteil (20) und mindestens ein den Handrückenteil (10) und den Handflächenteil (20) verbindendes erstes Befestigungsmittel (16,25) zur Befestigung der Daumenschiene an einer Hand,
**dadurch gekennzeichnet,**
**dass** die Hülse aus zwei offenen Daumenschalen (11,21) gebildet ist, deren Biegeradien flexibel sind und deren Ueberlappungsbereich stufenlos einstellbar und mindestens im Gebrauchszustand mittels mindestens eines zweiten Befestigungsmittels (27,28) fixierbar ist, wobei eine erste Daumenschale (11) am Handrückenteil (10) und eine zweite Daumenschale (21) am Handflächenteil (20) angeformt ist und
**dass** die Daumenschiene steife Bereiche zur Fixierung des Daumens in seiner Lage und flexible Bereiche zur Grösseneinstellung und Befestigung an der Hand aufweist.

2. Daumenschiene nach Anspruch 1, **dadurch gekennzeichnet, dass** jede der zwei Daumenschalen (11,21) im Bereich des Daumenfingerknochens maximal zu einem Viertel ihres Umfanges offen ist.

3. Daumenschiene nach einem der Ansprüche 1 oder 2, **dadurch gekennzeichnet,**
**dass** die erste Daumenschale (11) von der zweiten Daumenschale (21) mindestens teilweise umschlossen ist,
**dass** sie handrückenseitig einen vorstehenden Noppen (15) aufweist,
**dass** die zweite Daumenschale (21) auf einem handrückenseitigen Abschnitt einen Verstellschlitz (29) zur Aufnahme des Noppens (15) aufweist, und
**dass** der Biegeradius der zweiten Daumenschale (21) mittels mindestens eines Klettbandes (27) fixierbar ist.

4. Daumenschiene nach Anspruch 3, **dadurch gekennzeichnet, dass** an das mindestens eine Klettband (27) an einer ersten Längskante (23) der zweiten Daumenschale (21) angebracht ist und dass an einer zweiten Längskante (23') der zweiten Daumenschale (21) mindestens ein Längsschlitz (28) angeordnet ist zur Aufnahme des Klettbandes (27).

5. Daumenschiene nach Anspruch 1, **dadurch gekennzeichnet, dass** die erste Daumenschale (11) mindestens einen Einschnitt (14) zur Erhöhung der Flexibilität aufweist.

6. Daumenschiene nach Anspruch 1, **dadurch gekennzeichnet, dass** die erste Daumenschale (11) einen steifen Rücken (12) aufweist, welcher im Gebrauchszustand auf der vom Zeigefinger abgewandten Seite des Daumens zu liegen kommt, und dass die Bereiche beidseitig des Rückens (12) flexibel ausgebildet sind.

7. Daumenschiene nach Anspruch 1, **dadurch gekennzeichnet,**
**dass** die zweite Daumenschale (21) handflächenseitig flexibel ausgestaltet ist bis auf einen Bereich, welcher steif ausgebildet ist und sich vom Handflächenteil (20) über einen Sattel (24), welcher im Gebrauchszustand zwischen Daumen und Zeigefinger aufliegt, erstreckt und
**dass** der handrückenseitige Bereich der zweiten Daumenschale (21) steif ausgebildet ist.

8. Daumenschiene nach Anspruch 1, **dadurch gekennzeichnet, dass** das anatomisch vorgeformte Handflächenteil (20) bis auf Randbereiche steif ausgebildet ist.

9. Daumenschiene nach Anspruch 1, **dadurch gekennzeichnet, dass** am Handrücken- und/oder am Handflächenteil (10,20) eine Handgelenkmanschette angebracht ist.

10. Daumenschiene nach Anspruch 9, **dadurch gekennzeichnet, dass** die Handgelenkmanschette einstückig am Handrücken- und/oder am Handflächenteil (10,20) angeformt ist.

## Claims

1. A thumb splint for immobilising a thumb, with a sleeve for accommodating the thumb, with a hand-back part (10), an anatomically preformed palm part (20) and at least one first fastening means (16, 25) connecting the hand-back part (10) and the palm part (20), for fastening the thumb splint on a hand, **characterised in that**
the sleeve is formed of two open thumb shells (11, 21), whose bending radii are flexible and whose overlapping region is infinitely adjustable and at least in the condition of usage is fixable by way of at least one second fastening means (27, 28), wherein a first thumb shell (11) is formed on the hand-back part (10) and a second thumb shell (21) on the palm part (20) and that the thumb splint comprises stiff regions for fixing the thumb in its position and flexible regions for size adjustment and fastening on the hand.

2. A thumb splint according to claim 1, **characterised in that** each of the two thumb shells (11, 21) in the region of the thumb finger bone is open to maximally a quarter of its periphery.

3. A thumb splint according to claim 1 or 2, **characterised in that** the first thumb shell (11) is at least partly enclosed by the second thumb shell (21),that it on the side of the hand-back comprises a projecting nap (15),
that the second thumb shell (21) on a section on the hand-back side comprises an adjusting slot (29) for receiving the nap (15), and
that the bending radius of the second thumb shell (21) is fixable by way of at least one Velcro-type strip (27).

4. A thumb splint according to claim 3, **characterised in that** the at least one Velcro-type strip (27) is attached on a first longitudinal edge (23) of the second thumb shell (21) and that on a second longitudinal edge (23') of the second thumb shell (21) there is arranged at least one longitudinal slot (28) for receiving the Velcro-type strip (27).

5. A thumb splint according to claim 1, **characterised in that** the first thumb shell (11) comprises at least one incision (14) for increasing the flexibility.

6. A thumb splint according to claim 1, **characterised in that** the first thumb shell (11) has a stiff back (12) which in the condition of use comes to lie on the side of the thumb which is distant to the index finger, and that the regions on both sides of the back (12) are formed flexible.

7. A thumb splint according to claim 1, **characterised in that** the second thumb shell (21) on the side of the palm is formed flexible up to a region which is formed stiffly and which extends from the palm part (20) over a saddle (24) which in the condition of use rests between the thumb and index finger and that the hand-back-side region of the second thumb shell (21) is formed stiffly.

8. A thumb splint according to claim 1, **characterised in that** the anatomically preformed palm part (20) is formed stiffly up to the edge regions.

9. A thumb splint according to claim 1, **characterised in that** on the hand-back and/or palm part (10, 20) there is attached a wrist sleeve.

10. A thumb splint according to claim 9, **characterised in that** the hand wrist sleeve is formed as one piece on the hand-back and/or palm part (10, 20).

## Revendications

1. Attelle pour le pouce pour la mise au repos d'un pouce avec une gaine pour le maintien du pouce, une partie pour le revers de la main (10), une partie pour la paume de la main (20) pourvue d'une forme anatomique et au moins un premier moyen d'attache (16,25) reliant la partie pour le revers de la main (10) et la partie pour la paume de la main (20), pour la fixation de l'attelle pour le pouce à une main,
**caractérisée en ce**
**que** la gaine est formée de deux coquilles de pouce ouvertes (11,21), dont les rayons de courbure sont flexibles et dont la zone de recouvrement peut être ajustée en continu et peut être fixée au moins dans l'état d'utilisation au moyen d'au moins un deuxième moyen de fixation (27,28), tandis qu'une première coquille de pouce (11) est formée sur la partie pour le revers de la main (10) et une deuxième coquille de pouce (21) sur la partie pour la paume de la main (20) et
**que** l'attelle pour le pouce présente des zones rigides pour la fixation du pouce dans sa position et des zones souples pour l'ajustement en taille et la fixation à la main.

2. Attelle pour le pouce selon la revendication 1, **caractérisée en ce que** chacune des deux coquilles pour le pouce (11,21) est ouverte dans la zone de l'os du pouce d'au maximum un quart de son périmètre.

3. Attelle pour le pouce selon l'une des revendications 1 ou 2, **caractérisée en ce**
**que** la première coquille de pouce (11) est entourée au moins partiellement par la deuxième coquille de pouce (21),
**qu'**elle présente du côté du revers de la main un bouton saillant (15),
**que** la deuxième coquille de pouce (21) présente sur une section située du côté du revers de la main une fente de réglage (29) destinée à recevoir le bouton (15), et
**que** le rayon de courbure de la deuxième coquille de pouce (21) peut être fixée au moyen d'au moins une bande agrippante (27).

4. Attelle pour le pouce selon la revendication 3, **caractérisée en ce que** sur la ou les bandes agrippantes (27) sont prévues sur une première arête longitudinale (23) de la deuxième coquille de pouce (21) et que sur une deuxième arête longitudinale (23') de la deuxième coquille de pouce (21) est prévue au moins une fente longitudinale (28) pour la fixation de la bande agrippante (27).

5. Attelle pour le pouce selon la revendication 1, **caractérisée en ce que** la première coquille de pouce (11) présente au moins une encoche (14) pour augmenter la flexibilité.

6. Attelle pour le pouce selon la revendication 1, **caractérisée en ce que** la première coquille de pouce (11) présente un envers rigide (12), qui vient reposer en état d'utilisation sur la face du pouce opposée à l'index, et que les zones des deux côtés de l'envers sont flexibles.

7. Attelle pour le pouce selon la revendication 1, **caractérisée en ce que** la deuxième coquille de pouce (21) est agencée pour être flexible du côté de la paume de la main jusqu'à une zone qui est conçue comme étant rigide et s'étend à partir de la partie pour la paume de la main (20), sur un épaulement (24) qui à l'état d'utilisation repose entre le pouce et l'index, et que la zone située du côté du revers de la main de la deuxième coquille de pouce (21) est conçue comme étant rigide.

8. Attelle pour le pouce selon la revendication 1, **caractérisée en ce que** la partie pour la paume de la main (20) pourvue d'une forme anatomique est formée comme étant rigide jusqu'à des zones périphériques.

9. Attelle pour le pouce selon la revendication 1, **caractérisée en ce qu'**un manchon pour le poignet est disposé sur la partie pour le revers de la main et/ou pour la paume de la main (10,20).

10. Attelle pour le pouce selon la revendication 9, **caractérisée en ce que** le manchon pour le poignet est formé en une seule pièce sur la partie pour le revers de la main et/ou pour la paume de la main (10,20).
